# DEMANDE DE BREVET EUROPEEN

(11) **EP 0 534 874 A1**
(43) Date de publication de la demande: **31.03.1993**
(21) Numéro de dépôt: 92420326.8
(22) Date de dépôt: 23.09.1992
(51) Int. Cl.: A61B 5/103, A61B 19/00

(54) **Dispositif pour la mesure des amplitudes de mouvement entre deux vertèbres dans trois plans orthogonaux**

(30) Priorité: 24.09.1991 FR 9111964
(71) Demandeur: Graf, Henry, F-69006 Lyon (FR)
(72) Inventeur: Graf, Henry, F-69006 Lyon (FR)
(74) Mandataire: Karmin, Roger

(57) **Abrégé**

Le dispositif de mesure comprend deux éléments de réference (4, 5) associés respectivement de manière rigide à chaque vertèbre (1, 2) des moyens (19, 22) associés aux deux éléments de référence (5, 5) et permettant de mesurer l'angle de liberté des vertèbres considérées en torsion, c'est-à-dire dans un plan perpendiculaire à l'axe longitudinal de ces vertèbres et d'autres moyens (24, 27, 28, 30, 31, 33) associés auxdits éléments (4, 5) de l'angle de liberté des vertèbres en inflexion latérale, c'est-à-dire dans un plan (R) perpendiculaire celui (P) des apophyses épineuses en fonction d'une position données de flexion-extension desdites vertèbres, c'est-à-dire dans le plan (P) contenant les apophyses épineuses.

## Description

La présente invention est relative à un dispositif destiné à mesurer d'une part l'angle de liberté de deux vertèbres voisines en torsion et d'autre part l'angle de liberté de ces vertèbres en inflexion latérale par rapport à une position donnée en flexion-extension desdites vertèbres.

On considère dans la théorie que l'articulation intervertébrale comprend 6° de liberté, mais dans la pratique la liberté principale de l'articulation se situe en flexion-extension. Il existe de même, mais à moindre amplitude, des dégrés de liberté lors de la flexion latérale ou de la rotation horizontale intervertébrale.

Il faut noter également que ces deux mouvements sont habituellement couplés l'un à l'autre, c'est-à-dire que la flexion latérale entraîne obligatoirement un certain degré de rotation horizontale et vice versa.

Le problème de cette articulation intervertébrale lombaire est qu'elle subit rapidement des dommages entraînant une diminution de la qualité caoutchouteuse du disque. Cette détérioration aboutit à une plus grande souplesse de l'articulation, ce qui entraîne des mouvements plus amples tant en flexion-extension, mais également en inflexion latérale et en torsion.

La diminution de la qualité caoutchouteuse du disque entraîne une désorganisation complète des mouvements de l'articulation intervertébrale lombaire.

Il semble donc important, pour certaines décisions thérapeutiques, que l'on puisse mesurer à la fois l'amplitude de tous les mouvements intervertébraux et avoir une idée de la désorganisation du couplage des mouvements entre eux.

C'est à la solution de ce problème que s'est plus particulièrement attachée la présente invention.

La présente invention a pour but de concevoir un dispositif de mesure susceptible d'indiquer, à tout moment et en toute position, les rotations tri-dimensionnelles d'une vertèbre par rapport à l'autre. Ce dispositif permettra en particulier d'apprécier les mouvements d'inflexion et de rotation intervertébrale à quelques endroits de la flexion-extension intervertébrale.

Les mesures obtenues par ce dispositif permettront d'apporter des arguments décisionnels pour certaines positions thérapeutiques et ceci "in vivo", pendant les interventions. Le dispositif de mesure pourra être utilisé en dehors de la chirurgie en prenant appui en percutanée sur les épineuses intervertébrales. Pendant une opération, le dispositif de mesure trouvera appui sur des vis qui seront placées dans les pedicules de la vertèbre.

Le dispositif pour la mesure des amplitudes de deux vertèbres suivant la présente invention comprend deux éléments de référence associés respectivement de manière rigide à chaque vertèbre, des moyens associés à deux éléments de référence et permettant de mesurer l'angle de liberté des vertèbres considérées en torsion, c'est-à-dire dans un plan perpendiculaire à l'axe longitudinal de ces vertèbres, et d'autres moyens associés auxdits éléments permettant de mesurer l'angle de liberté des vertèbres en inflexion latérale, c'est-à-dire dans un plan perpendiculaire à celui des apophyses épineuses en fonction d'une position donnée de flexion-extension desdites vertèbres, c'est-à-dire dans le plan contenant les apophyses épineuses.

Le dessin annexé, donné à titre d'exemple, permettra de mieux comprendre l'invention, les caractéristiques qu'elle présente et les avantages qu'elle est susceptible de procurer :
Fig. 1 est une vue en perspective du dispositif suivant l'invention.
Fig. 2 est une vue de dessus de ce dispositif.
Fig. 3 est une vue semblable à celle de fig. 1, mais illustrant deux vertèbres voisines déplacées angulairement l'une par rapport à l'autre dans le sens d'une cyphose.
Fig. 4 montre la mesure de l'angle de torsion des deux vertèbres à partir de leur position illustrée en fig. 3.
Fig. 5 est une vue semblable à celle de fig. 2, mais illustrant la mesure de l'angle de déplacement des deux vertèbres en inflexion latérale à partir de leur position de fig. 3.
Fig. 6 est une vue en perspective d'une variante du dispositif de fig. 1.

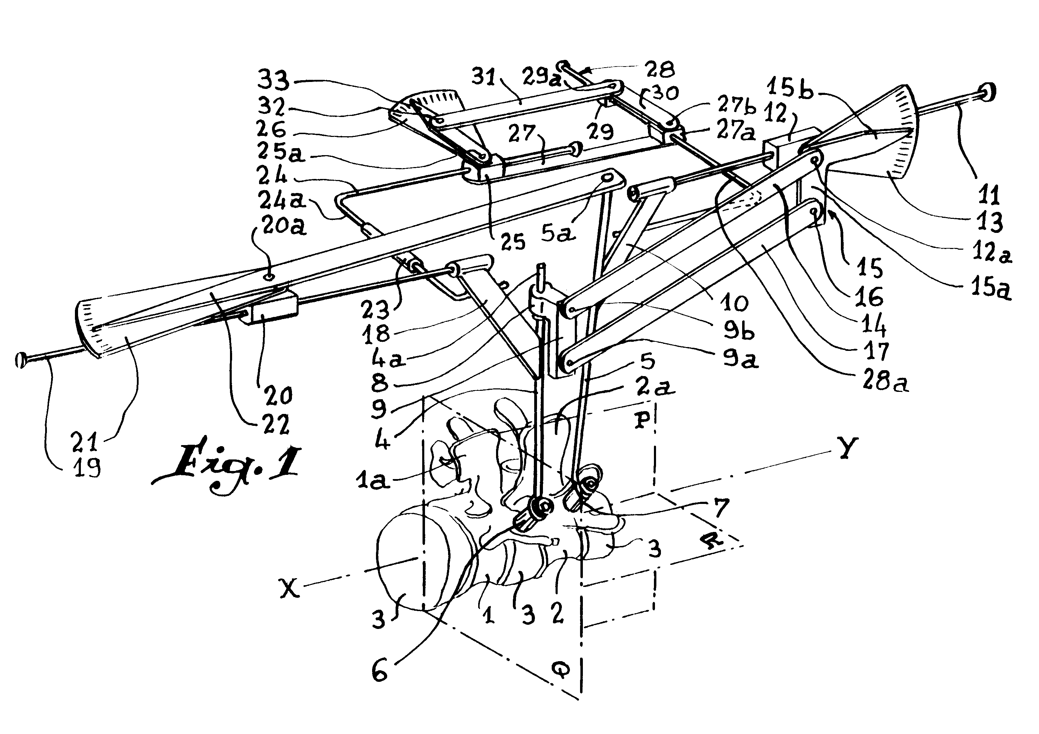

On a illustré en fig. 1 deux vertèbres voisines 1, 2 séparées à la manière usuelle par un disque 3. On a référencé X-Y l'axe longitudinal des vertèbres et on a tracé un plan P contenant cet axe ainsi que les apophyses épineuses 1a, 2a des vertèbres 1 et 2. Dans cette figure, on a également représenté un plan transversal Q passant par le disque 3 et orthogonal à l'axe X, Y ainsi qu'au plan P. Un troisième plan R contenant l'axe X, Y a aussi été représenté en fig. 1, son orientation étant orthogonale aux deux plans P et Q.

Dans la suite des présentes, on appellera flexion-extension le mouvement des vertèbres en position par exemple de cyphose dans laquelle l'axe X-Y devient courbe en restant dans le plan P. On appellera torsion la rotation d'une vertèbre par rapport à l'autre dans des plans parallèles au plan Q. Enfin, on appellera inflexion latérale le mouvement des vertèbres l'une par rapport à l'autre tel que l'axe X-Y se courbe en restant dans le plan R.

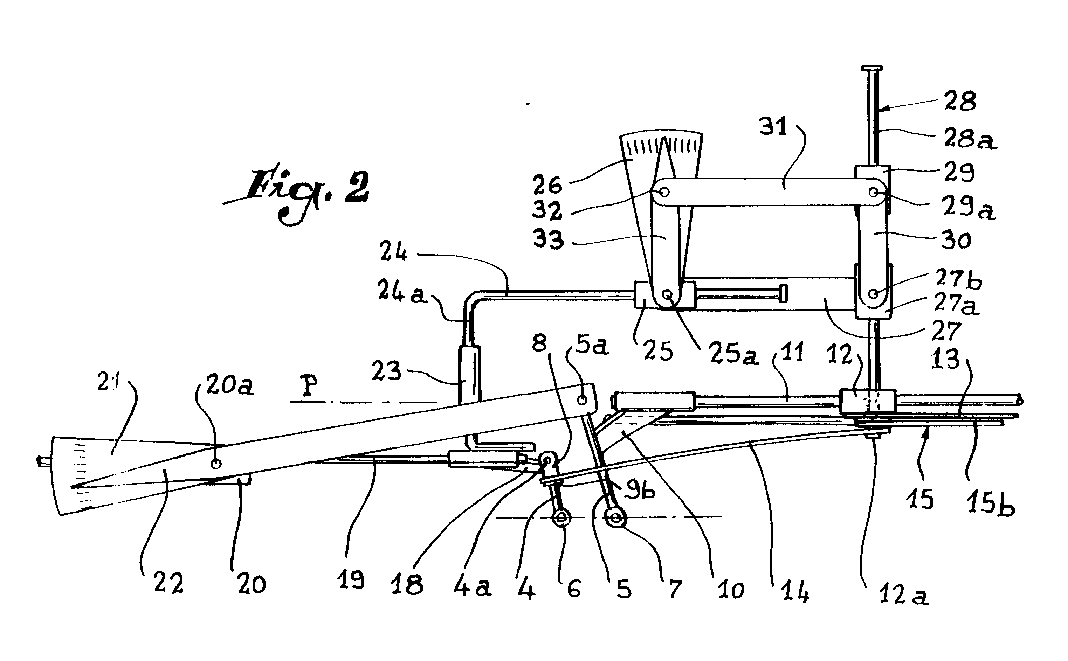

Le dispositif suivant l'invention comprend tout d'abord deux colonnettes 4, 5 dont l'une des extrémités est fixée respectivement à chacune des vertèbres 1 et 2 par tous moyens appropriés par exemple des implants ou des vis 6, 7. Les deux colonnettes s'élèvent de manière légèrement oblique afin que l'extrémité libre de la colonnette 4 se trouve décalée latéralement par rapport au plan P dont on a représenté la trace en fig. 2, tandis que l'extrémité libre de la colonnette 5 se trouve pratiquement contenue dans ce plan.

L'extrémité libre de la colonnette 4 comprend un embout 4a à plus faible diamètre qui s'engage librement dans le trou d'un dé 8 comportant une barrette 9 s'étendant parallèlement à la colonne 4. Cette barrette comporte deux pivots 9a, 9b.

La partie supérieure de la colonnette 5 est solidaire d'un profilé plat oblique 10 auquel est fixée une tige 11 s'étendant en gros parallèlement à l'axe P (voir fig. 2). Sur cette tige est monté à coulissement libre un chariot 12 auquel est fixé un segment de cercle formant cadran 13 convenablement gradué et parallèle au plan P. Sur sa face latérale, le chariot 12 comporte un pivot 12a parallèle à ceux 9a, 9b de la barrette 9. Une biellette souple 14 est articulée aux pivots 9b, 12a. Autour de ce dernier pivote le sommet d'une équerre 15 dont l'une 15a des branches, parallèle à la barrette 9 et de même longueur, est pourvue d'un axe 16 d'articulation pour l'une des extrémités d'une biellette souple 17 dont l'autre extrémité est articulée au pivot 9a. L'autre branche 15b de l'équerre 15 constitue une aiguille qui se déplace devant la portion graduée du cadran 13. Les deux biellettes 14, 17, la barrette 9 et la branche 15a de l'aiguille 15 forment un parallèlogramme déformable.

La partie supérieure de la colonnette 4 située immédiatement en dessous du dé 8 est pourvue d'un profilé plat oblique 18 solidaire d'une tige 19 s'étendant perpendiculairement à la colonnette 4 et pratiquement de manière parallèle à la tige 11, comme illustré en fig. 2. Un chariot 20 est monté à coulissement libre le long de la tige 19. Ce chariot porte un cadran 21 convenablement gradué disposé parallèlement au plan R ainsi qu'un axe 20a autour duquel est articulée une aiguille 22 dont l'extrémité opposée à celle située devant la graduation du cadran 21 est articulée autour du bout libre 5a de la colonnette 5.

Un palier 23 en gros parallèle au plan R est fixé au profilé 18, son orientation étant perpendiculaire au plan formé par cette colonnette 4 et la tige 19. Dans ce palier est montée à rotation libre l'extrémité de l'une des branches 24a d'une broche en équerre 24 dont la seconde branche, parallèle à la tige 19, reçoit à coulissement libre un dé 25 portant un cadran 26 gradué et orienté en gros parallèlement au plan R. Ce dé est également solidaire de l'une des extrémités d'une barrette 27 dont l'autre extrémité comporte une tête 27a traversée à coulissement libre par une tringle 28 solidaire de la deuxième colonnette 5. Cette tringle présente la forme d'une équerre dont la branche 28a qui coopère avec la tête 27a se trouve perpendiculaire au plan déterminé par la colonnette 5 et la tige 11. La branche 28a de la tringle 28 reçoit à coulissement libre un bloc 29. Ce dernier, le dé 25 et la tête 27a portent respectivement des pivots 29a, 25a et 27b. Entre les pivots 27b et 29a est monté un levier 30. Un levier 31 est articulé par l'une de ses extrémités au pivot 29a et par son autre extrémité à un axe 32 solidaire d'une aiguille 33 dont l'extrémité opposée à sa partie indicatrice est articulée autour du pivot 25a. Ainsi la barrette 27, les leviers 30 et 31 et l'aiguille 33 forment-ils une parallèlogramme déformable.

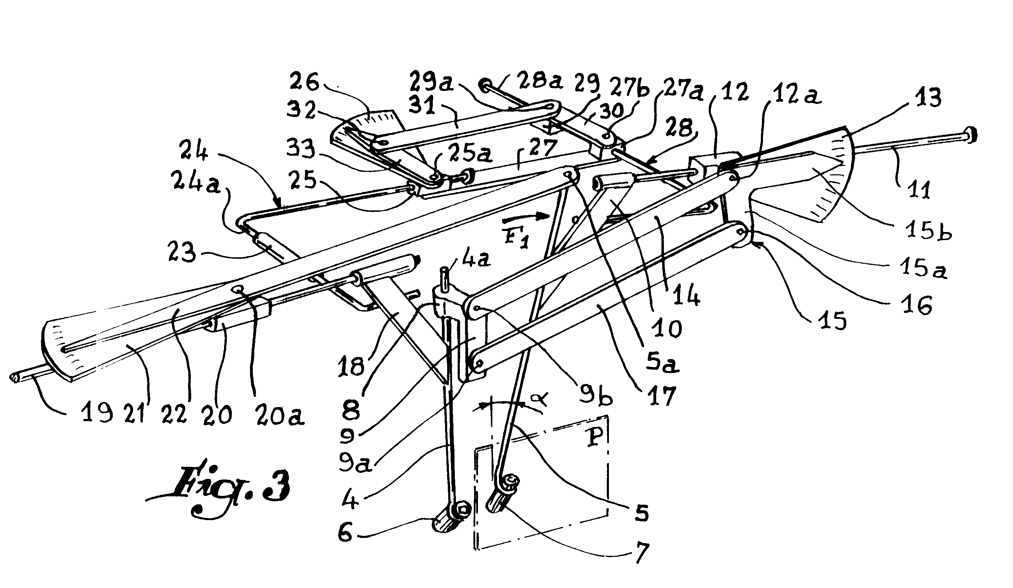

Le fonctionnement est le suivant : au cours de la mesure de l'amplitude du débattement des deux vertèbres 1 et 2 dans le sens de la flexion-extension, la courbure des vertèbres dans ce sens entraîne la création d'un angle α (fig. 3) entre les colonnettes 4 et 5, de telle sorte que l'extrémité 5a de la colonnette 5 s'éloigne de celle 4a de la colonnette 4 (flèche F₁), lesdites colonnettes étant déplacées dans des plans parallèles au plan P. Ce déplacement provoque le coulissement du chariot 12 le long de la tige 11, de telle sorte que le parallélogramme déformable formé par la barrette 9, les deux biellettes souples 14, 17 et la branche 15a de l'aiguille 15 se déforme, en entraînant le déplacement de l'extrémité de l'aiguille 15 par rapport à la graduation du cadran 13 pour indiquer la valeur de l'angle α . Bien entendu, ce déplacement entraîne aussi un coulissement du chariot 20 par rapport à la tige 19, mais sans modification notable de la position de l'aiguille 22. Simultanément, la tringle 28 bascule en direction des vertèbres, ce qui provoque une rotation de la broche 24 dans le palier 23. Par suite du parallèlisme entre les tiges 11 et 19, la position de l'aiguille 33 par rapport à la graduation du cadran 26 ne varie pas de manière apparente.

A partir de toute position relative de flexion-extension des deux vertèbres 1 et 2, il est possible de mesurer les amplitudes en rotation et en inflexion latérale des deux vertèbres.

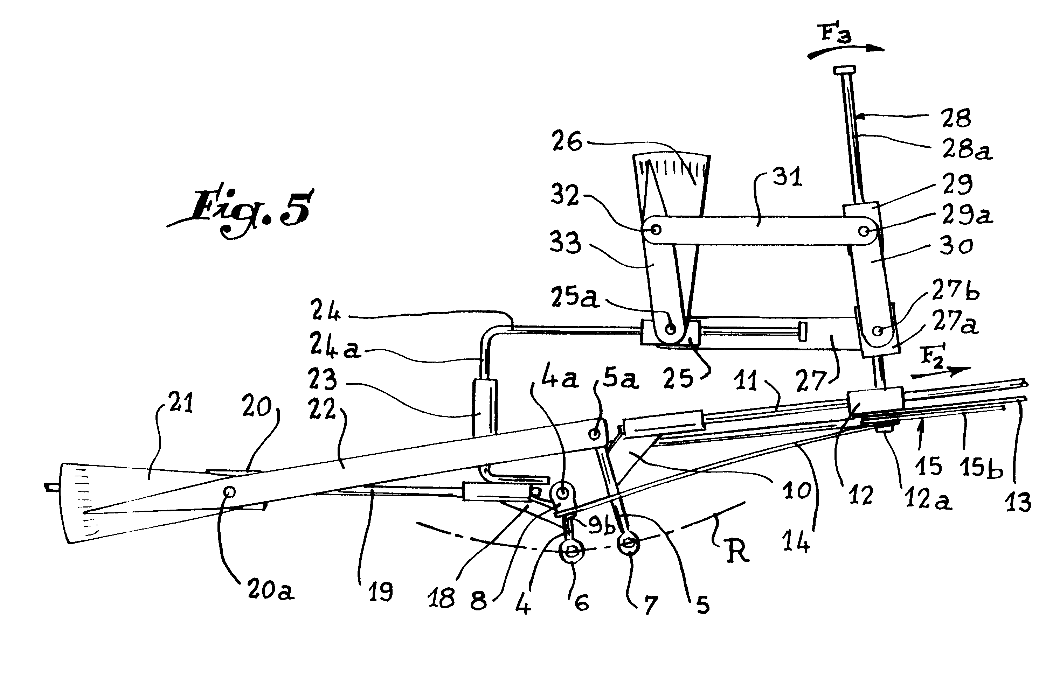

Si les vertèbres sont déplacées en inflexion latérale, c'est-à-dire en donnant à l'axe X-Y une courbure dans le plan R, les deux colonnettes 4 et 5 opèrent des rotations complexes, de telle sorte que d'une première part le chariot 20 glisse le long de la tige 19 du fait de l'éloignement des deux colonnettes 4 et 5, et d'une seconde part la tringle 28 change de position relative vis-à-vis de la broche 24 en s'éloignant d'elle suivant la flèche F2 de fig. 5 tout en effectuant un basculement suivant la flèche F3. Dans ces conditions, le parallèlogramme déformable 27, 30, 31, 33 se déforme et l'extrémité de l'aiguille 33 se déplace par rapport à la graduation du cadran 26 pour indiquer l'angle de débattement maximal des deux vertèbres en inflexion latérale.

On peut également mesurer l'angle maximal de rotation d'une vertèbre par rapport à l'autre dans le plan Q. Le déplacement relatif des deux colonnettes 4 et 5 dans ce plan Q entraîne, dans le cas de la flexion latérale, un éloignement des deux extrémités 4a et 5a desdites colonnettes qui provoque d'une part le coulissement du chariot 20 sur la tige 19 et une rotation subséquente de l'aiguille 22 dont l'extrémité se déplace devant la graduation du cadran 21 pour indiquer par exemple l'amplitude maximale de liberté des deux vertèbres dans le sens de la rotation.

On comprendra dans ces conditions l'utilité de prévoir les biellettes 14 et 17 souples pour qu'elles puissent suivre sans déformation les mouvements relatifs des colonnettes 4 et 5 en rotation et en inflexion latérale.

Bien entendu des jauges de proximité ou des potentiomètres pourraient être prévus au niveau des axes 12a du chariot 12, 20a du chariot 20 et 25a du dé 25 pour mesurer les angles de débattement au moyen d'un convertisseur électronique à affichage numérique ou appareil analogue auquel les jauges ou potentiomètres seraient branchés.

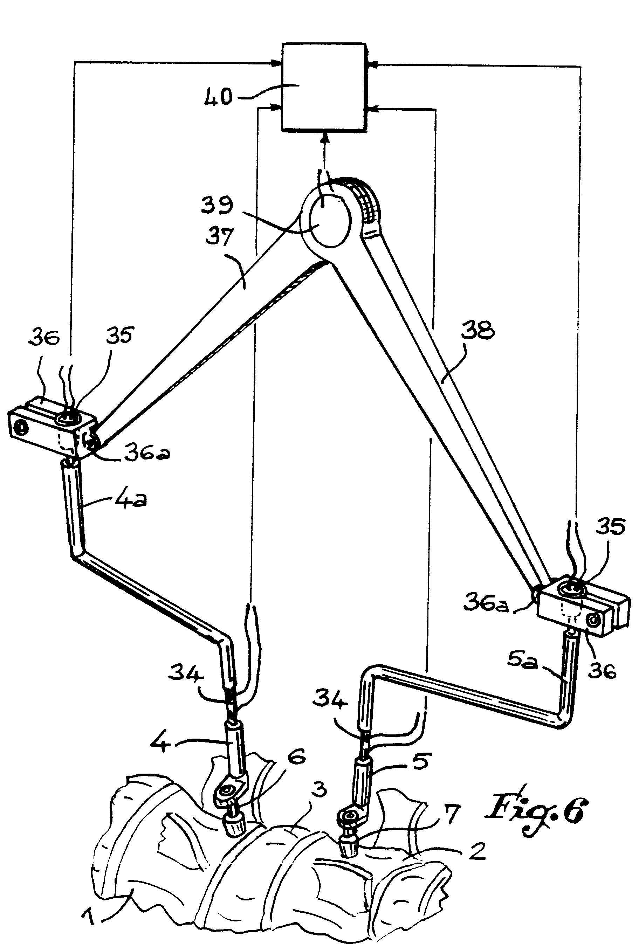

On a illustré en fig. 6 une variante d'exécution du dispositif de mesure suivant l'invention.

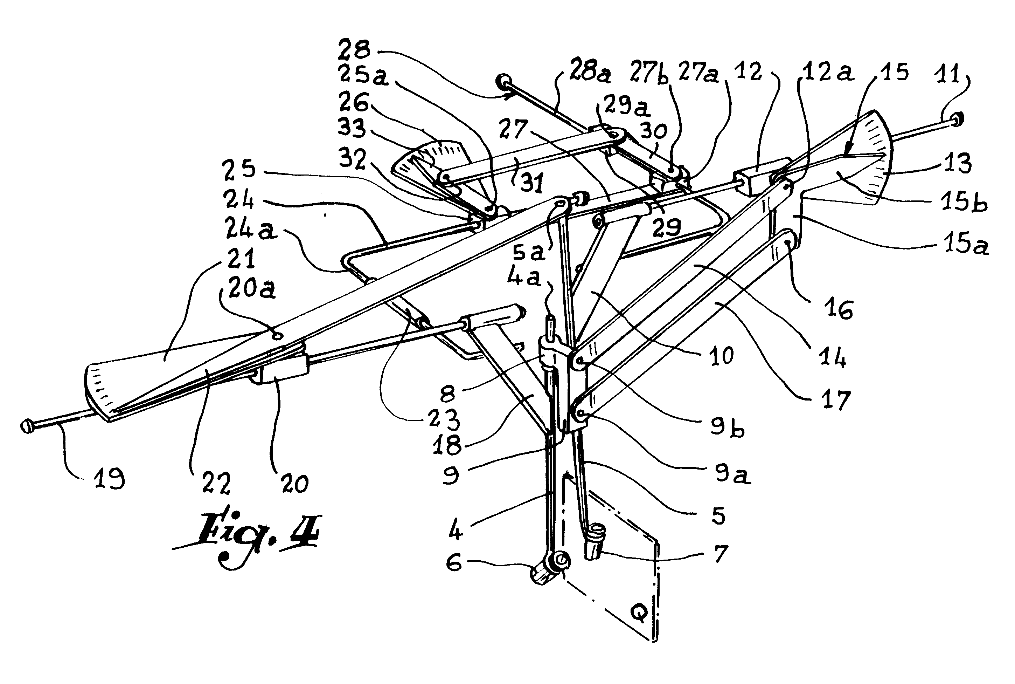

Le dispositif illustré en fig. 1 à 6 permet la mesure des angles de torsion, d'inflexion latérale et de flexion-extension de vertébrés voisines mais sans référence à la force appliquée sur ces vertèbres pour les déplacer dans les trois directions précitées.

Au contraire, conformément au dispositif de fig. 6, il est possible de mesurer les angles de torsion et de flexion-extension des vertèbres 1 et 2 en fonction de la force exercée sur celles-ci par l'intermédiaire des colonnettes 4 et 5 qui comportent chacune une jauge de contrainte 34. Cette jauge est placée à proximité de la fixation des colonnettes 4 et 5 par rapport aux vis ou implants 6, 7 solidarisés aux vertèbres 1 et 2. Les colonnettes 4 et 5 sont en réalité constituées par des tiges coudées deux fois en S et qui se trouvent disposées en gros dans le plan P contenant l'axe longitudinal X-Y du rachis.

Les extrémités 4a, 5a des tiges qui se trouvent en gros parallèles aux vis 6 et 7 portent à leurs extrémités une jauge de proximité rotative 35 propre à tourner dans un dé 36. Les faces en vis-à-vis des deux dés 36 comportent chacune une chappe 36a dans laquelle est articulée l'une des extrémités d'une biellette 37, respectivement 38. L'extrémité opposée de la biellette 38 est solidaire d'une jauge de proximité 39 tournant par rapport à un axe fixé à l'extrémité correspondante de l'autre biellette 37. Ainsi les biellettes 37 et 38 forment une espèce de compas dont les branches sont disposées dans le plan P.

Les jauges 34, 35 et 39 sont connectées à un convertisseur électronique à affichage numérique 40 propre à afficher les angles de torsion et de flexion-extension des vertèbres 1 et 2 en fonction de l'effort appliqué sur celles-ci en déplaçant les tiges 4 et 5, soit en torsion, soit en flexion-extension.

Cette mesure s'effectue donc par exemple en torsion en déplaçant les deux tiges 4 et 5 dans deux directions opposées perpendiculairement au plan P. Ce déplacement provoque la rotation des jauges de proximité 35 par rapport aux dés 36, les dés restant dans le plan P du fait de leur liaison aux biellettes 37 et 38.

Si l'on désire effectuer une mesure de l'angle de flexion-extension, les tiges 4, 5 sont déplacées dans le plan P en direction opposée ce qui provoque le déplacement des deux dés 36 en s'éloignant l'un de l'autre si bien qu'alors c'est la jauge 39 qui mesure la variation de l'angle des biellettes.

Il va de soi que les jauges de proximité peuvent être remplacées par des potentiomètres rotatifs sans sortir du cadre de l'invention ou par tout autre dispositif analogue.

## Revendications

1. Dispositif pour la mesure des amplitudes de deux vertèbres (1, 2) dans trois plans orthogonaux (P, Q, R), caractérisé en ce qu'il comprend deux éléments de référence (4, 5) associés respectivement de manière rigide à chaque vertèbre (1, 2), des moyens (19, 22, 34, 35, 36, 37, 38) associés aux deux éléments de référence (4, 5) et permettant de mesurer l'angle de liberté des vertèbres considérées en torsion, c'est-à-dire dans un plan perpendiculaire à l'axe longitudinal de ces vertèbres, et d'autres moyens (8, 9, 14, 15, 17, 34, 36, 37, 38, 39) de mesure de l'angle de basculement des vertèbres (1, 2) en flexion-extension.

2. Dispositif suivant la revendication 1, caractérisé en ce qu'il comporte aussi des moyens (24, 27, 28, 30, 31, 33) associés auxdits éléments (4, 5) et permettant de mesurer l'angle de liberté des vertèbres en inflexion latérale, c'est-à-dire dans un plan (R) perpendiculaire à celui (P) des apophyses épineuses en fonction d'une position donnée de flexion-extension desdites vertèbres, c'est-à-dire dans le plan (P) contenant les apophyses épineuses.

3. Dispositif suivant la revendication 1, caractérisé en ce que les éléments de référence sont deux colonnettes (4, 5) dont les extrémités inférieures sont ancrées dans les vertèbres (1, 2), la première colonnette (4) comportant un dé (8) monté à rotation libre autour de l'axe longitudinal de ladite colonnette comportant une barrette (9) parallèle à cette dernière et qui est pourvue de deux pivots (9a, 9b) espacés auxquels sont articulées les extrémités de deux biellettes souples (14, 17) formant avec l'une des branches (15a) d'une aiguille (15) un parallélogramme déformable dont l'un des axes d'articulation (12a) est porté par un chariot (12) monté à coulissement libre par rapport à une tige (11) fixée perpendiculairement à la deuxième colonnette (5), tandis que le chariot (12) porte un cadran (13) devant lequel se déplace la branche libre de l'aiguille (15).

4. Dispositif suivant la revendication 1, caractérisé en ce que l'élément de référence (4) est une première colonnette (4) solidaire d'une tige (19) orientée perpendiculairement à cette colonnette et sur laquelle tige (19) est monté à coulissement libre un chariot (20) portant un cadran (21) et un pivot (20a) sur lequel est articulée une aiguille rectiligne (22) également articulée par rapport à l'extrémité libre (5a) de la seconde colonnette (5) qui constitue le second élément de référence.

5. Dispositif suivant la revendication 2, caractérisé en ce qu'un palier (23) est fixé à l'élément de référence (4) constitué par une première colonnette (4) en étant orienté perpendiculairement au plan formé par cette colonnette et la tige (19) qui en est solidaire et dans lequel est monté à rotation libre l'extrémité d'une des branches (24a) d'une broche en équerre (24) dont la seconde branche, parallèle à la tige (19), reçoit à coulissement libre un dé (25) portant un cadran (26), ledit dé (25) étant solidaire d'une des extrémités d'une barrette (27) dont l'autre extrémité comporte une tête (27a) portant un pivot (27b) et qui est traversée à coulissement libre par une tringle (28) solidaire du deuxième élément 5 affectant la forme d'une colonnette (5) et orientée perpendiculairement au plan que cette colonnette détermine avec sa tige fixe (11), ladite tringle recevant à coulissement libre un bloc (29) pourvu d'un pivot (29a), tandis que des leviers (30, 31) s'articulent aux pivots (27b, 29a) de la tête (27a) et du bloc (29) et à une aiguille (33) articulée au dé (25) pour former avec la barrette (9) un parallélogramme déformable.

6. Dispositif suivant la revendication 1, caractérisé en ce que les moyens de mesurer les angles de liberté en torsion et en inflexion latérale sont constitués par des jauges de proximité rotatives branchées à un convertisseur électronique à affichage numérique.

7. Dispositif suivant la revendication 2, caractérisé en ce que les moyens de mesurer l'angle de liberté des vertèbres en inflexion latérale sont constitués par des jauges de proximité rotatives branchées à un convertisseur électronique à affichage numérique.

8. Dispositif suivant la revendication 1, caractérisé en ce que les deux éléments de référence (4, 5) associés aux vertèbres (1, 2) sont chacun pourvu d'une jauge de contrainte (34), les extrémités (4a, 5a) desdits éléments de référence étant associés à une jauge de proximité rotative (35) tournant dans un dé (36) auquel est articulé une biellette, les deux biellettes (37, 38) disposées dans le plan (P) étant associées à un axe de pivotement associé à une jauge de proximité rotative (39) solidaire d'une des biellettes, lesdites jauges de contrainte et de proximité étant connectées à un convertisseur électronique (40) à affichage digital des angles de torsion et de flexion extension des vertèbres en fonction de l'effort appliqué sur celles-ci.
